# EUROPEAN PATENT APPLICATION

(11) **EP 3 127 511 A1**
(43) Date of publication of application: **08.02.2017**
(21) Application number: 15179749.5
(22) Date of filing: 04.08.2015
(51) Int. Cl.: A61F 2/52

(54) **METHOD OF MAKING A BREAST COSMESIS**

(71) Applicant: Dorset Orthopaedic Company Ltd., Ringwood, Hampshire BH24 3PB (GB)
(72) Inventor: Watts, Robert John, Ringwood,, Hampshire, BH24 3PB (GB)
(74) Representative: Plöger, Jan Manfred

(57) **Abstract**

The invention relates to a method of making a breast cosmesis (44) comprising the steps of:
(a) manufacturing a breast skin gel (20) having a gel colour that corresponds to the breast skin colour,
(b) manufacturing a breast skin element (34) from the breast skin gel (20) using a standardized mould (28),
(c) capturing an areola contour of an areola of the patient,
(d) manufacturing a model cast with a depression of a negative form of the areola contour,
(e) manufacturing an elastic areola element having the areola contour using the model cast, and
(f) fixing the areola element to the breast skin element (20) so that a breast cosmesis is provided.

## Description

The invention relates to a method of making a breast skin cover for breast cosmesis. According to a second aspect, the invention relates to a breast cosmesis and to a kit comprised of a first gel cushion, a first breast skin element that is sized to fit around the first gel breast form, and an areola element.

After breast surgery, such as a partial or complete mastectomy, some women choose to wear a breast cosmesis. It is an important aspect for these women that when they hug someone the other person does not get the impression that the woman is wearing a breast cosmesis.

It is also desirable that the breast cosmesis blends with the woman's skin colour and body shape as well as possible so that the woman can wear e.g. a swimsuit or a semi-see-through dress. However, it has been very work-intensive in the past to build high-quality breast cosmesis.

The objective of the present invention is to provide an improved method of making a breast skin cover for an off the shelf gel breast form.

The invention solves the problem by means of a method of making a breast skin cover for a breast cosmesis comprising the steps of (a) capturing a breast skin colour of a breast skin of a patient, in particular of breast skin of the contralateral breast, (b) manufacturing a breast skin gel having a gel colour that corresponds to the patient's breast skin colour, (c) manufacturing a breast skin element from the breast skin gel using a standardized mould, (d) capturing an areola contour of an areola of the patient, the areola in particular having the same contour as the contralateral areola, (e) manufacturing a model cast with a depression of the negative form of the areola contour, (f) manufacturing an elastic areola element with the areola contour using the model cast, and (g) fixing or attaching the areola element to the breast skin element, so that the breast cosmesis is provided. Step (a) is optional.

According to a second aspect, the invention solves the problem by means of a breast cosmesis comprised of a breast skin element for covering a gel cushion on at least one side, and an areola element, wherein the areola element has a smaller hardness, i.e. is less hard, than the breast skin element. According to a third aspect, the invention solves the problem by means of a kit according to claim 11.

It is an advantage of the present invention that the breast cosmesis is very realistic and easy to manufacture at the same time. The invention is based on the idea that the shape of the breast does not differ as much between women as the areola and the nipple do in respect to colour and contour. It is therefore sufficient to provide an individualized areola element and an individual breast skin colour, whereas the shape of the breast skin element can be chosen from a fixed set of standardized moulds, based upon existing off the shelf gel breast forms already used in this sector.

In the present description, the contralateral breast should be understood as the breast which is opposite to the breast that is to be replaced by the breast cosmesis.

A standardized mould is to be understood as a mould that is not custom-made for the patient. In particular, the standardized mould is part of a set of pre-fabricated moulds. Preferably this set of moulds comprises of 50 moulds or less. It is an advantage if the breast skin gel is a silicone gel. The colour of the gel is chosen so that breast skin element's colour matches the patient's breast skin colour, in particular the colour of the patient's contralateral breast.

According to a preferred embodiment, the method comprises the following steps: capturing an areola colour of the areola of the patient, and manufacturing an areola skin gel having a gel colour that corresponds to the areola colour, wherein the elastic areola element is manufactured from the areola skin gel so that the areola element corresponds to the patient's areola's colour and contour, in particular to the patient's contralateral areola's colour and its contour.

In other words, the areola element is individualized so that it conforms to the patient's areola's contour, i.e. shape, and colour. It is possible to use the contralateral areola's contour and colour. As an alternative, the areola colour and contour, and optionally the nipples' colour and contour, of the breast that is going to be removed in a surgical operation later can be used. The areola and the nipple are a highly characteristic feature of a woman's breast. By capturing the areolas colour and contour, and optionally the nipples' colour and contour, the resulting areola element matches the original closely and gives the wearer the impression that the breast cosmesis is highly individualized, even though its shape need not match the patient's contralateral breast shape precisely.

It is preferred that the breast skin element is manufactured by pour moulding. The silicone is vacuum treated and then poured into a two part mould. The vacuum treatment of the silicone gel removes unwanted bubbles so that the skin element has a smooth surface. The pour moulding can be carried out at ambient air pressure. It is an additional advantage that pour moulding results in a smooth breast skin element, as there is no need for ducts in the mould.

According to a preferred embodiment, the method comprises the step of painting, in particular air-brushing, the breast element in at least a transition area surrounding the areola element. The areola element may be made from a different kind of gel as the breast skin element. This could result in different appearances. Painting the area surrounding the areola element and preferably parts of the areola element as well, even if these differences disappear so that the breast cosmesis looks as if it was made from one mould only. In other words, the resulting breast cosmesis has a particularly natural appearance. The nipple may be painted in order to more closely match the patient's contralateral breast's nipple.

The painting of the breast element may include painting of any characteristics such as freckles, veins and/or moles. This yields a particularly faithful reproduction of the breast skin.

According to a preferred embodiment, the areola element is manufactured so that is has an areola element hardness that is smaller than the skin hardness of the breast skin element. In other words, the areola element material that the areola element is made from has a lesser hardness than the breast skin element material that the breast skin element is made from. The hardness may be the Shore-A-hardness. It is a surprising finding that these kind of breast cosmesis have a particularly natural haptic impression, because the nipple region is usually stiffer than the surrounding skin.

It is preferred that the areola element has a Shore-A hardness of 10 to 20. Alternatively or additionally, the breast skin element has a Shore-A hardness of 20 to 30.

According to a preferred embodiment, the method comprises the following steps: providing a gel breast form and fixing the breast cosmesis to the gel breast form so that a breast cosmesis is provided.

The aspects of the teachings of the present invention, and arrangements embodying those teachings, are hereafter described by way of an illustrated example with reference to the accompanying drawings, in which:
- Figure 1: is a schematic view of a patient wherein an areola contour of an areola of the patient is being captured,
- Figure 2: comprising the Figures 2a, 2b, 2c and 2d, shows a standardized mould for manufacturing a breast skin element as part of an embodiment of the present invention,
- Figure 3: with the Figures 3a, 3b, and 3c show the upper mould element, the breast skin element and a breast cosmesis comprising the breast skin element and a breast cosmesis according to the present invention comprising a breast skin element and a gel cushion,
- Figure 4: with the sub-Figures 4a, 4b, 4c and 4d shows how the areola element is produced and fixed to the breast skin element.

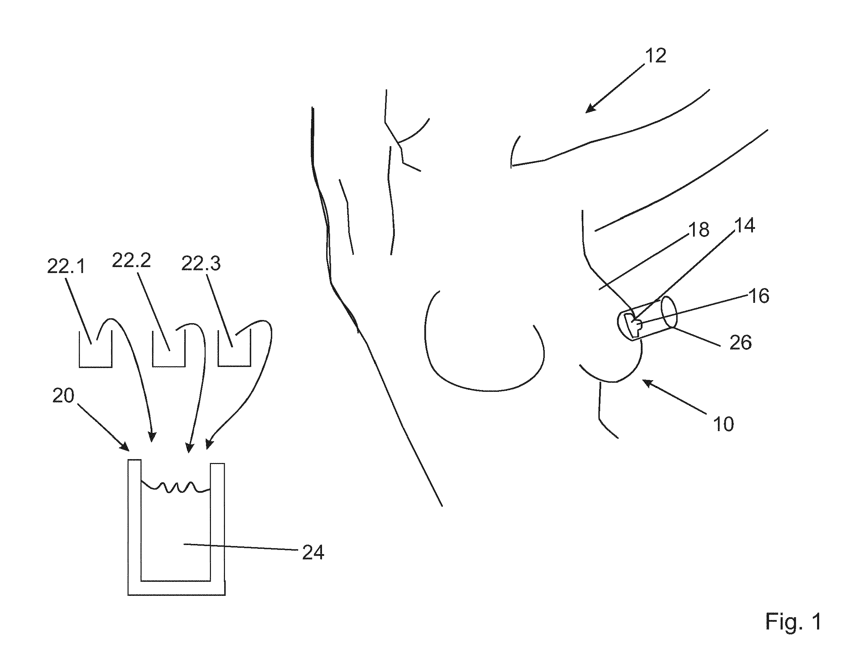

Figure 1 shows a breast 10 of a patient 12, the breast 10 comprising an areola 14 and a nipple 16.

In a first step of a method according to the present invention, a breast skin silicone gel 20 is provided that has a colour corresponding to the breast skin colour of a breast skin 18. This may be done by adding pigments 22.1, 22.2, 22.3 to a silicon precursor gel 24.

An areola contour of the areola 14 including the nipple 16 is captured by means of a beaker 26 containing Life Form Silicone, manufactured by Mouldlife.

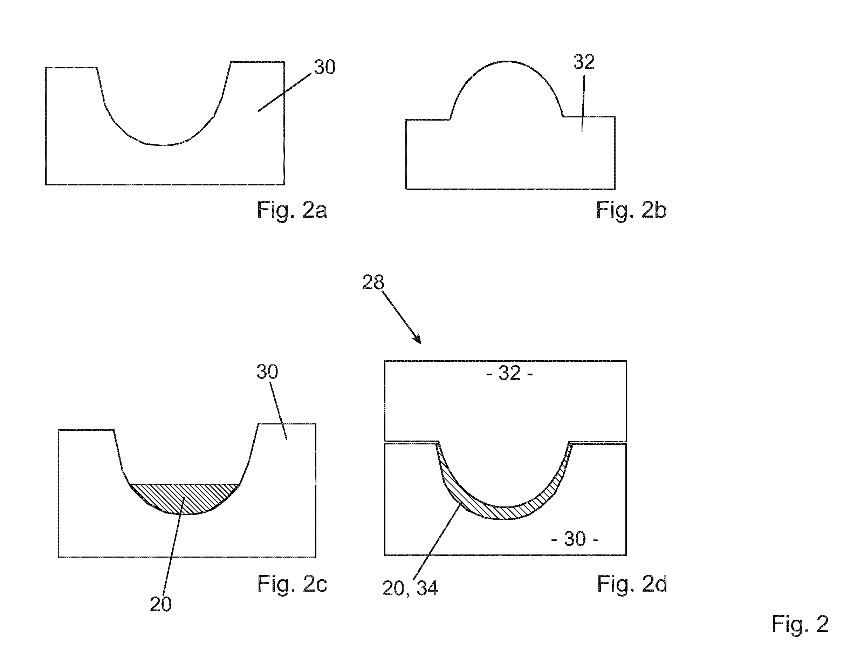

In a further step, a standardized mould 28 (cf. Figure 2d) is chosen according to the size of the breast 10 and its shape. An areola skin gel is mixed in the same way as the breast skin gel 20, so that the areola skin gel has a colour that corresponds to the colour of the areola 14. It should be noted that the patient 12 had her right breast removed in a surgical procedure, so that the breast 10 is the contralateral breast.

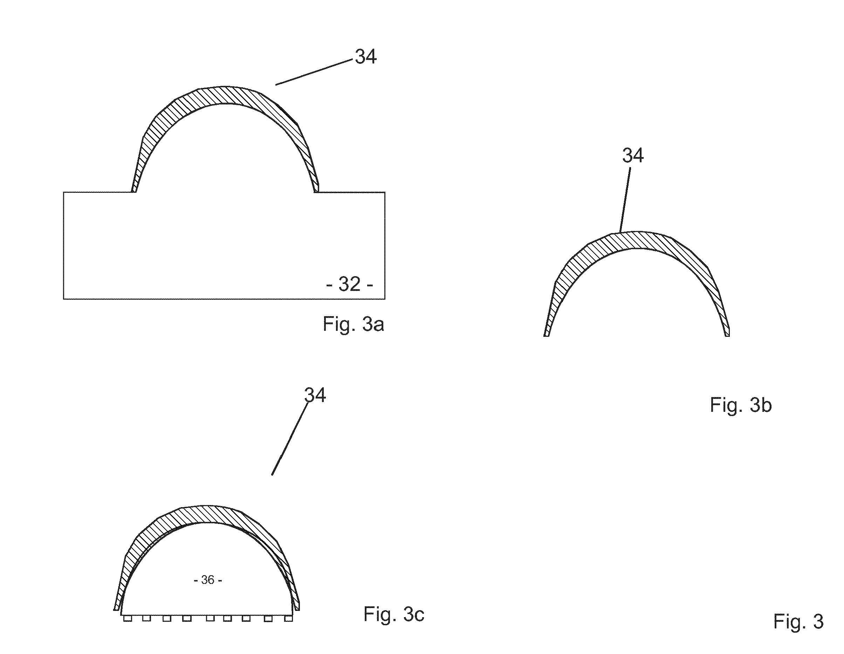

Figure 2 a shows a lower mould element 30 that corresponds to an upper mould element 32 (cf. Figure 2b). The breast skin gel 20 is poured into the lower mould element (30) as can be seen in Figure 2c. The upper mould element 32 is then lowered onto the lower mould element 30, as shown in Figure 2d. After setting, the breast skin gel 20 forms a breast skin element 34. Figure 3a shows the breast skin element 34 on the upper mould element 32. Figure 3b shows the breast skin element 34 after removing it from the upper mould element.

Figure 3c shows a gel breast form 36 and the breast skin element 34 that is sized to fit around the gel breast form 36. It should be noted that the upper mould element 32 has a contour that is chosen to adapt to the contour of a gel breast form 36. In other words, the size of the gel cushion 36 is chosen first and the upper mould element 32 is then chosen to conform to the gel cushions shape.

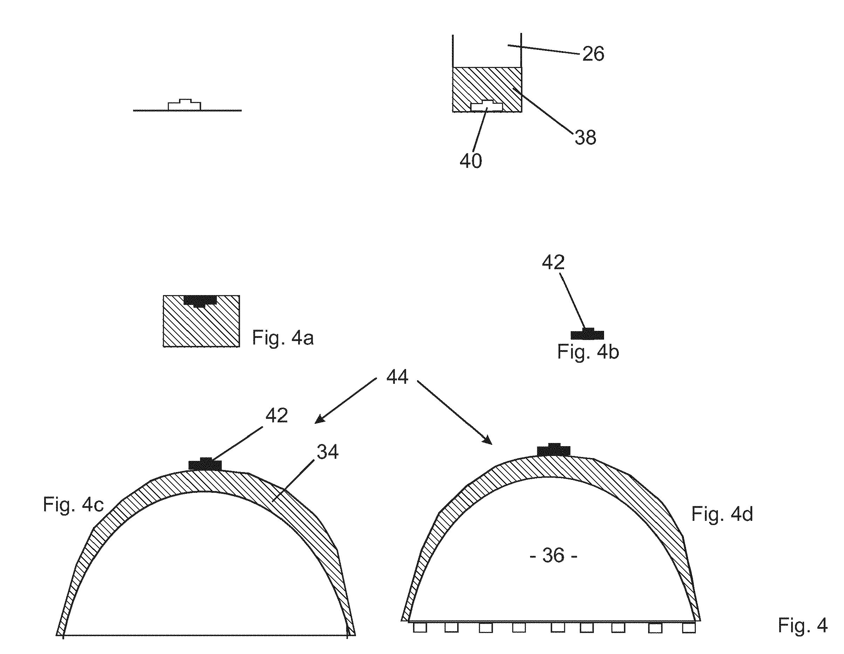

Figure 4a shows the beaker 26 with the set casting material 38 that shows an indentation 40 in which the areola skin gel is poured to form an areola skin element 42 after setting.

Figure 4c shows the areola skin element 42 that has been glued to the breast skin element 34, thus forming a breast cosmesis 44. The breast cosmesis 44 may than be fixed to the gel cushion 36, as can been seen in Figure 4d.

### Reference list

- 10: breast
- 12: patient
- 14: areola
- 16: nipple
- 18: breast skin

- 20: breast skin gel
- 22: pigment
- 24: precursor gel
- 26: beaker
- 28: mould

- 30: lower mould element
- 32: upper mould element
- 34: breast skin element
- 36: gel breast form
- 38: casting material

- 40: indentation
- 42: areola skin element
- 44: breast cosmesis

## Claims

1. A method of making a breast cosmesis (44) comprising the steps of:
(a) manufacturing a breast skin gel (20) having a gel colour that corresponds to the breast skin colour,
(b) manufacturing a breast skin element (34) from the breast skin gel (20) using a standardized mould (28),
(c) capturing an areola contour of an areola of the patient,
(d) manufacturing a model cast with a depression of a negative form of the areola contour,
(e) manufacturing an elastic areola element having the areola contour using the model cast, and
(f) fixing the areola element to the breast skin element (20) so that a breast cosmesis is provided.

2. The method according to claim 1, comprising the following steps:
- capturing an areola colour of the areola (14) and a nipple colour of a nipple (16) of the patient, and
- manufacturing an areola skin gel (20) having a gel colour that corresponds to the areola colour,
- wherein the elastic areola element is manufactured from the areola skin gel (20) so that the areola element corresponds to the patient's areola's colour and contour.

3. The method according to any of the proceeding claims, **characterized in that**
the breast skin element (34) is manufactured by pour moulding.

4. The method according to any of the proceeding claims, comprising the step of
painting, in particular air-brushing, the breast element in at least a transition area surrounding the areola element.

5. The method according to claim 4, **characterized in that** the step of painting, in particular air-brushing, the breast element skin (34) includes painting characteristics such as freckles, veins, and moles onto the breast skin element (34).

6. The method according to any of the proceeding claims, **characterized in that** the areola element is manufactured such that it has an areola element hardness that is less than the skin hardness of the breast skin element (34).

7. The method according to any of the proceeding claims, **characterized in that**
- the areola element has a Shore-A hardness of 10 to 20 and/or
- the breast skin element (34) has a Shore-A hardness of 20 to 30.

8. The method according to any of the proceeding claims, comprising the following steps:
- providing a gel breast form (36) and
- fixing the breast cosmesis to the gel breast form (36) so that a breast cosmesis is provided.

9. A breast cosmesis comprising
(a) a breast skin element for covering a gel breast form (36) on at least one side, and
(b) an areola element,
**characterized in that**
(c) the areola element has less hardness than the breast skin element (34).

10. The breast cosmesis according to claim 9, **characterized in that** the areola element is separately fixed to the breast skin element.

11. Kit comprising
(a) a first gel breast form,
(b) a first breast skin element that is sized to fit around the first gel breast form, and
(c) an areola element,
**characterized by** comprising
(d) a second gel breast form, wherein the first breast skin element is sized to fit around the second gel breast form and/or a second breast skin element that is sized to fit around the first gel breast form.

12. Kit according to claim 11, **characterized in that** the second gel breast form has lower density than the first gel breast form.

13. Kit according to claim 11 or 12, **characterized in that** the first gel breast form has a density of 0.95 to 1.05 g/cm³ and/or
the second gel breast form has a density of less than 0.95 g/cm³.
